# EUROPÄISCHE PATENTSCHRIFT

(11) **EP 3 134 944 B1**
(45) Veröffentlichungstag und Bekanntmachung des Hinweises auf die Patenterteilung: **03.07.2019**
(21) Anmeldenummer: 15719448.1
(22) Anmeldetag: 22.04.2015
(51) Int. Cl.: H01R 13/648, H01R 31/06, A61N 1/14, H01R 13/44, H01R 13/506

(54) **KOPPLUNGSELEMENT FÜR EIN ELEKTRISCH BETRIEBENES GERÄT UND DAMIT AUSGEBILDETES SYSTEM**
COUPLING ELEMENT FOR AN ELECTRICALLY OPERATED DEVICE AND SYSTEM FORMED THEREWITH
ÉLÉMENT DE COUPLAGE POUR APPAREIL À FONCTIONNEMENT ÉLECTRIQUE ET SYSTÈME ÉQUIPÉ DE CE DERNIER

(30) Priorität: 24.04.2014 DE 202014101937 U
(43) Veröffentlichungstag der Anmeldung: 01.03.2017
(73) Patentinhaber: Zumtobel Lighting GmbH, 6850 Dornbirn (AT)
(72) Erfinder: BADER, Martin, A-6850 Dornbirn (AT); GALLER, Gerhard, A-6900 Bregenz (AT); REIN, Thomas, A-6850 Dornbirn (AT)
(74) Vertreter: Kiwit, Benedikt
(86) Internationale Anmeldenummer: PCT/EP2015/058695
(87) Internationale Veröffentlichungsnummer: WO 2015/162168

(56) Entgegenhaltungen:
- WO-A1-02/19475
- WO-A1-2008/016874
- DE-A1- 4 415 910
- GB-A- 886 464
- US-A- 4 313 148
- US-A1- 2004 014 339

## Beschreibung

Die Erfindung betrifft ein Element zum elektrischen Koppeln für ein elektrisch betriebenes Gerät, das für die Aufnahme oder Halterung elektronischer Komponenten vorgesehen ist. Diese elektronischen Komponenten müssen vor elektrostatischen Entladungen geschützt werden. Insbesondere kann es sich bei dem elektrisch betriebenen Gerät um eine Leuchte handeln, in der LEDs als Leuchtmittel zum Einsatz kommen. Die Erfindung betrifft ferner ein mit solch einem Kopplungselement gebildetes System und ein damit verbundenes Verfahren.

In der Beleuchtungstechnologie kommen in den letzten Jahren vermehrt LEDs als Leuchtmittel zum Einsatz. Der Grund liegt insbesondere in ihrer langen Lebensdauer, geringen Energieaufnahme und der Möglichkeit, beispielsweise deren Helligkeit und/oder Leuchtfarbe dynamisch verändern zu können. Im Gegensatz zu klassischen Leuchtmitteln, wie Leuchtstofflampen oder Glühbirnen, sind LED-Module allerdings sensibler gegenüber äußeren Einflüssen. Insbesondere sogenannte elektrostatische Entladungen (electrostatic discharges - ESD) können zu einer Beschädigung der Leuchtmittel führen. Diese sind möglicherweise im ersten Augenblick nicht unmittelbar erkennbar, können aber zu einem späteren Zeitpunkt auftreten und im Extremfall zu einem vollständigen Ausfall der Leuchte führen.

Um unbeabsichtigte elektrostatische Entladungen zu vermeiden, wird von Herstellern solcher LED-Module üblicherweise vorgeschrieben, dass deren elektronischen Bauteile unter besonderen Schutzmaßnahmen zu behandeln sind. Diese Schutzmaßnahmen sind während der gesamten Herstellung, Montage sowie auch während der späteren Wartung der Leuchten einzuhalten. Für das Einhalten dieser ESD-Schutzmaßnahmen auch nach der Installation bzw. Montage solch einer Leuchte gibt es Lösungen.

Diese Lösungen erfordern, die betreffende Person vor Ort erden zu müssen. Vor Ort gibt es kaum Möglichkeiten beispielsweise in Form einer elektrischen Kopplung der Person mit dem Untergrund (Boden) in Form einer Bodenmatte oder dergleichen. Insbesondere wenn die Person auf einen Aufzug oder eine Leiter steigen muss, um an die Leuchte an einer Decke zu gelangen, ist das Erden an sich ein Problem. Daher wurde vorgeschlagen, die Person über das Gerät selbst zu erden, nämlich über einen dafür extra vorgesehenen Erdungsanschluss.

Damit ist zwar das Problem des Erdens gelöst. Allerdings kann es insbesondere bei Geräten mit metallischen Gehäusen dazu kommen, dass das Metall unter Strom steht. Berührt die Person das Metall, kann sie einen Stromschlag oder dergleichen erhalten und Verletzungen davontragen. Es könnte beispielsweise aufgrund von Defekten im Gerät auftreten, dass das Gehäuse des Geräts unter Strom steht. In diesem Fall wäre aufgrund möglicher hoher Spannungen und insbesondere zu hoher elektrischer Ströme das Herstellen einer elektrischen Verbindung zwischen Gerät und Person über ein handelsübliches Erdungsband gefährlich.

US 2004/014339 A1 offenbart eine Vorrichtung zum Erde von elektrischen Geräte während Reparatur- oder Wartungsarbeiten.

Der Erfindung liegt daher die Aufgabe zugrunde, eine Möglichkeit zu schaffen, jederzeit Arbeiten an elektrisch betriebenen Geräten durchführen zu können, ohne dabei Gefahren aufgrund möglicher elektrostatischer Aufladung und eines etwaigen Unterstromstehens solcher Geräte einzugehen.

Die Aufgabe wird durch den Gegenstand der unabhängigen Ansprüche gelöst. Vorteilhafte Weiterbildungen sind in den Unteransprüchen angegeben.

Erfindungsgemäß ist ein Kopplungselement gemäß dem unabhängigen Anspruch 1 vorgesehen, das einen ersten Kopplungsabschnitt aufweist. Der erste Kopplungsabschnitt ist gestaltet, mit einem ersten Erdungsanschluss an einen zweiten Erdungsanschluss eines zweiten Kopplungsabschnitts eines elektrisch betriebenen Geräts elektrisch angeschlossen zu werden. Der erste Erdungsanschluss ist mithin Bestandteil des ersten Kopplungsabschnitts. Das Gerät benötigt eine elektrische Energieversorgung. Der zweite Kopplungsabschnitt ist daher zum elektrischen Anschluss dieser Energieversorgung für das Gerät ausgebildet. Das Kopplungselement weist ferner ein Erdungsmittel mit einem ersten Anschlussabschnitt auf. Der erste Anschlussabschnitt ist mit dem ersten Erdungsanschluss elektrisch gekoppelt und zudem ausgebildet, mit einer Person unmittelbar elektrisch gekoppelt zu werden. Das Erdungsmittel dient also der Erdung einer Person und ist vorzugsweise als Antistatikband ausgebildet, das weithin verfügbar und preiswert ist. Der erste Anschlussabschnitt stellt dann die Handmanschette dar, über die solch ein Band üblicherweise verfügt. Diese Lösung hat zum einen den Vorteil, dass die Energieversorgung erst einmal vom Gerät elektrisch getrennt werden muss, bevor sich die Person (nur am Gerät) erden kann, nämlich über das Kopplungselement und dessen ersten Erdungsanschluss. Dadurch wird erreicht, dass ein etwaig vorhandenes, aus einem stromleitenden Material bestehendes Teil des Geräts, wie eine Abdeckung oder ein Gehäuse, das für die Person zugänglich ist, nicht mehr unter Strom stehen kann, wenn sich die Person mit dem Gerät elektrisch koppelt. Ein anderer Vorteil besteht darin, dass das Gerät selbst nicht über einen gesonderten Erdungsanschluss für das genannte Erdungsmittel verfügen muss. Die Erfindung ist also universell einsetzbar. Und da als zweiter Kopplungsabschnitt des Geräts für die Erdung der Person ein Geräteanschluss für eine externe Energieversorgung genutzt wird, ist es dadurch möglich, die Erdung über sowieso im Gerät vorhandene Sicherheitsmittel zu realisieren.

Das Gerät kann beispielsweise ein LED-Lichtband sein, bei dem an bestimmten Positionen Steckbuchsen vorgesehen sind, welche das Anschließen eines Konverters zur Stromversorgung von LED-Modulen des Lichtbands ermöglichen. Vorzugsweise ist vorgesehen, dass alternativ oder zusätzlich zu einem an dem Leuchtengehäuse vorgesehenen Anschluss zur Herstellung einer elektrischen Kopplung mit einem Erdungsband eine Buchse als zweiter Kopplungsabschnitt genutzt wird, die eigentlich zur Stromversorgung des Konverters genutzt wird. Das Kopplungselement umfasst dann einen speziellen Stecker, der mit der Buchse mechanisch und elektrisch koppelbar gestaltet ist. Dieser Stecker ist vorzugsweise derart ausgeführt, dass er einen einzigen Steckkontakt aufweist, der mit dem Erdungsanschluss der Buchse bzw. einem dementsprechenden geerdeten Abschnitt der Buchse in Eingriff gebracht wird. Dieser einzelne Erdungsstecker kann dann wiederum mit einem Druckknopfanschluss versehen sein, der das Anschließen des Erdungsbands ermöglicht, oder mit einem Erdungsband elektrisch und mechanisch gekoppelt ist.

Das Erdungsmittel kann in das Kopplungselement integriert sein. Dies verhindert, dass die Person das Erdungsmittel an das Gerät anschließen kann, sofern dieses über einen entsprechenden Erdungsanschluss verfügt. Dadurch ist die Gefahr gebannt, dass sich die Person über das Erdungsmittel mit dem Gerät elektrisch koppeln und mit einem stromführenden Teil des Geräts in Berührung kommen könnte.

Der erste Anschlussabschnitt ist vorzugsweise über einen Entladewiderstand mit dem ersten Erdungsanschluss elektrisch gekoppelt. Dadurch können für Personen gefährliche Ströme vermieden werden, die insbesondere bei hohen Spannungen ohne Zwischenwiderstand fließen könnten. Eine Person, die Reparatur- oder Wartungsarbeiten beispielsweise an einer Leuchte mit darin befindlichen LED-Modulen durchführen muss, kann sich also über ein entsprechendes Erdungsmittel mit dem zweiten Kopplungsabschnitt beispielsweise in Form eines üblicherweise vorhandenen, mit einem Nullleiteranschluss versehenen elektrischen Anschluss der Leuchte elektrisch koppeln. Über den Entladewiderstand und den ohnehin vorhandenen Erdungsanschluss der Leuchte kann dann ein definiertes Entladen der Person erfolgen. Ist nunmehr die Person dauerhaft in der beschriebenen Weise mit der Leuchte elektrisch gekoppelt, können während der Arbeiten keine elektrostatischen Entladungen mehr auftreten und der vorgenannte ESD-Schutz ist gewährleistet; und dies mit der Sicherheit des Schutzes vor nunmehr nicht mehr vorhandenen, möglicherweise stromführenden Teilen. Ist der Anschlussabschnitt gestaltet, mit einem Erdungsmittel elektrisch gekoppelt zu werden, ermöglicht das Vorsehen des Widerstands zwischen erstem Anschlussabschnitt und erstem Erdungsanschluss den einfachen Anschluss einer einfachen elektrischen Leitung; ein Antistatikband mit gegebenenfalls integriertem Entladewiderstand ist nicht erforderlich.

Das Erdungsmittel weist vorzugsweise einen zweiten Anschlussabschnitt auf. Dieser ist gestaltet, mit einem dritten Anschlussabschnitt des Kopplungselements lösbar elektrisch gekoppelt zu werden, der mit dem ersten Erdungsanschluss elektrisch gekoppelt ist. D. h. die Person muss sich zum Kopplungselement nicht noch ein Erdungsmittel kaufen, wenn sie bereits eines hat.

Der zweite und dritte Anschlussabschnitt können mittels zweier miteinander korrespondierender und vorzugsweise ineinander steckbarer Druckknopfanschlüsse gebildet sein, wie dies bei üblichen Antistatikbändern der Fall ist. D. h. mit der Erfindung können weiterhin vorhandene Erdungsmittel genutzt werden, was die Kosten senken hilft.

Die erfindungsgemäße Lösung basiert also auf einem System, wie es in ähnlicher Weise auch an ESD-geschützten Arbeitsplätzen zum Einsatz kommt. Um sicherzustellen, dass die an einem derartigen Arbeitsplatz aktive Person nicht elektrostatisch aufgeladen wird, wird diese üblicherweise über ein Erdungskabel als Erdungsmittel geerdet. Dieses Erdungskabel wird beispielsweise über ein Handgelenkband an der Person befestigt und so mit dieser elektrisch gekoppelt. Ferner ist sie mit einem geerdeten Bereich des Arbeitsplatzes elektrisch gekoppelt, sodass die Person dauerhaft über das Erdungskabel entladen werden kann. Ein elektrostatisches Aufladen der Person während der Arbeit wird auf diesem Wege verhindert.

Jedes der vorgenannten Kopplungselemente ist zumindest zweiteilig ausgebildet. D. h. es weist ein erstes und ein zweites Anschlussteil auf. Das erste Anschlussteil weist den vorgenannten ersten Kopplungsabschnitt, einen dritten Kopplungsabschnitt und einen Verbindungsabschnitt auf. Das zweite Anschlussteil hat einen vierten Kopplungsabschnitt. Zum einen ist der Verbindungsabschnitt mit dem ersten und dritten Kopplungsabschnitt derart gekoppelt, und zum anderen ist der dritte Kopplungsabschnitt derart mit dem vierten Kopplungsabschnitt elektrisch koppelbar gestaltet, dass bei einem elektrischen Anschließen des zweiten Anschlussteils über das erste Anschlussteil bzw. dessen ersten Kopplungsabschnitt an das Gerät der erste Erdungsanschluss mit dem zweiten Erdungsanschluss elektrisch gekoppelt ist. Eines der Anschlussteile oder beide kann/können als Adapter fungieren, da deren Anschlusscharakteristik bzw. -konfiguration eine andere sein kann als die zum Anschluss zumindest an das Gerät. Der dritte und der vierte Kopplungsabschnitt entkoppeln die Anschlusskonfiguration zum Gerät von etwaig anderen vorhandenen Anschlusskonfigurationen. Beispielsweise kann das zweite Anschlussteil den vorgenannten Druckknopfanschluss aufweisen oder gar eine Kopplungsmöglichkeit beispielsweise zu einer externen Energieversorgung aufweisen. Dadurch muss nur noch eine Art des zweiten Anschlussteils vorgesehen werden. Für jede Art von Anschluss an das Gerät muss jeweils nur noch ein gesondertes, nämlich ein erstes, Anschlussteil vorgesehen werden. Dies ermöglicht für das zweite Anschlussteil die Möglichkeit der Standardisierung. Die Anpassung an das jeweilige Gerät erfolgt über erste Anschlussteile, die mit ihren ersten Kopplungsabschnitten an den jeweiligen zweiten Kopplungsabschnitt des jeweiligen Geräts angepasst sind. Das zweite Anschlussteil kann hinsichtlich der Anschlusskonfiguration völlig anders ausgebildet sein.

Der zweite Kopplungsabschnitt ist vorzugsweise als Stecker oder Buchse ausgebildet. Der mit dem zweiten Kopplungsabschnitt elektrisch zu koppelnde erste Kopplungsabschnitt des Kopplungselements bzw., wenn vorhanden, dritte Kopplungsabschnitt des ersten Anschlusselements ist als Buchse bzw. Stecker ausgebildet. Dieser zu koppelnde Kopplungsabschnitt ist so ausgebildet, dass er und der zweite Kopplungsabschnitt derart ineinander steckbar sind, dass dadurch automatisch der zweite Erdungsanschluss mit dem ersten Erdungsanschluss bzw. dem elektrischen Verbindungsabschnitt elektrisch gekoppelt ist. Somit können der erste und zweite Kupplungsabschnitt beide beispielsweise identisch ausgebildet sein, sodass sie nicht direkt miteinander gekoppelt werden können. Dies erhöht die Freiheitsgrade in der Gestaltung des Kopplungselements. Ferner ist es dadurch möglich, das Kopplungselement über ein oder mehrere zueinander verschiedene erste Anschlusselemente sowohl an einen als Stecker als auch als Buchse oder in sonstiger Weise ausgebildeten zweiten Kopplungsabschnitt des Geräts anschließen und damit elektrisch koppeln zu können.

Ist der dritte Kopplungsabschnitt als Stecker oder Buchse ausgebildet, ist der vierte Kopplungsabschnitt dementsprechend als Buchse bzw. Stecker ausgebildet, und zwar so, dass der dritte und vierte Kopplungsabschnitt derart ineinander steckbar sind, dass dadurch automatisch der erste Erdungsanschluss mit dem elektrischen Verbindungsabschnitt elektrisch gekoppelt ist. Es entsteht mithin eine übliche und einfach zu bedienende SteckerBuchsen-Kombination.

Ein erfindungsgemäßes System sieht eines der vorgenannten Geräte, also eines mit dem vorgenannten zweiten Kopplungsabschnitt, vor. Der zweite Kopplungsanschluss kann dem direkten Anschließen einer externen Energieversorgung oder einer Durchverdrahtung zur Energieversorgung dienen. Zudem sieht das System eines der vorgenannten Kopplungselemente vor. Durch die Erfindung entsteht mithin ein System mit sehr wenig Teilen. Dies macht die Benutzung einfach.

Vorzugsweise weist das Kopplungselement solch eines Systems ferner einen fünften Kopplungsabschnitt auf. Dieser Kopplungsabschnitt ist gemäß dem zweiten Kopplungsabschnitt des Geräts ausgebildet, weist also die gleiche Form auf wie der zweite Kopplungsabschnitt. Somit weist dieser Kopplungsabschnitt einen nunmehr dritten Erdungsanschluss auf. Der dritte Erdungsanschluss ist erfindungsgemäß mit dem ersten Erdungsanschluss elektrisch gekoppelt. Dies ermöglicht, das Kopplungselement zwischen Gerät und dessen Energieversorgung schalten zu können. Dadurch kann der Erdungsanschluss bzw. Nullleiter der Energieversorgung genutzt werden, die Person zu erden. Das Gerät benötigt also keinen zusätzlichen Erdungsanschluss als den, der ohnehin aufgrund des Energieversorgungsanschlusses (= zweiter Kopplungsabschnitt) vorhanden ist.

Der fünfte Kopplungsabschnitt ist vorzugsweise ferner gestaltet, Stromanschlüsse des zweiten Kopplungsabschnitts nach außen in Bezug auf das Gerät elektrisch zu isolieren. Wird das Kopplungselement zwischen Energieversorgung und Gerät geschaltet, kann dadurch keine Energie mehr in das Gerät eingeleitet werden, was sonst zu den vorgenannten Gefahren für die Person führen könnte. Im einfachsten Fall sind die beim zweiten Kopplungsabschnitt vorhandenen Stromanschlüsse beim fünften Kopplungsabschnitt zwar vorhanden, aber aus einem elektrisch nicht leitfähigen Material wie Kunststoff gebildet. D. h. die Montage bleibt weiterhin sehr einfach, und die Person muss nicht darauf achten, ob die Energieversorgung zugeschaltet ist oder nicht.

Die Erfindung ermöglicht trotz verbesserter Sicherheit ein sehr einfaches Verfahren zum Durchführen von Reparatur- oder Wartungsarbeiten an einem elektrisch betriebenen Gerät. Dazu ist erfindungsgemäß eines der vorgenannten Systeme, also mit Kopplungselement, vorgesehen. Das Verfahren weist als ersten Schritt das Lösen des elektrischen Anschlusses einer elektrischen Energieversorgung von dem Kopplungsgegenelement des Geräts. Damit ist das Gerät im Idealfall selbst stromlos. Danach erfolgt ein Schritt eines elektrischen Anschließens des ersten Kopplungsabschnitts des Kopplungselements, sodass dessen erster Erdungsanschluss an den zweiten Erdungsanschluss des Kopplungsgegenelements elektrisch angeschlossen ist. Damit sind etwaige stromführende Teile für die Person nicht mehr gefährlich. Daraufhin folgt ein Schritt eines elektrischen Koppelns der Person mit dem Erdungsmittel, sodass nunmehr auch die Elektronik des Geräts geschützt ist, nämlich vor möglichen statischen Aufladungen beispielsweise der Person.

Danach folgt nun der eigentliche Schritt des Durchführens von Tätigkeiten am Gerät, wie der Austausch defekter oder routinemäßig auszutauschender Teile des Geräts. Nach Abschluss dieser Tätigkeiten folgt ein Schritt eines elektrischen Trennens des Kopplungselements vom zweiten Kopplungsabschnitt des Geräts und eines Schritts eines elektrischen Wiederanschließens der Energieversorgung an den wieder frei gewordenen, zweiten Kopplungsabschnitt des Geräts. Damit ist das Gerät wieder betriebsbereit. Das Verfahren bleibt also weiterhin sehr einfach und dennoch sicher sowohl für die Person als auch für das Gerät.

Ist der vorgenannte Anschlussabschnitt ausgebildet, lösbar mit einem Erdungsmittel elektrisch gekoppelt zu werden, weist das Verfahren einen zusätzlichen Schritt eines elektrischen Koppelns des Erdungsmittels mit dem Anschlussabschnitt auf. Dieser Schritt erfolgt erfindungsgemäß unmittelbar vor oder nach dem Schritt des elektrischen Koppelns der Person mit dem Erdungsmittel. D. h. auch bei einem separat ausgebildeten Erdungsmittel bleibt das Verfahren sehr einfach, ohne Einbußen in der Sicherheit einzugehen.

Im Fall des vorgenannten Konverters muss ein Elektriker zum Durchführen von Wartungsarbeiten also zunächst den Stecker des Konverters aus der Buchse herausziehen und den speziellen Stecker (= Kopplungselement) in die Buchse einstecken. Anschließend kann wird das Erdungsband mit dem Druckknopfanschluss des speziellen Steckers, wenn vorhanden, gekoppelt werden, sodass hierdurch ein Potentialausgleich zum Gerät durchgeführt wird. Gleichzeitig wird durch den speziellen Stecker die Gefahr ausgeschlossen, dass der Elektriker versehentlich ein stromführendes Element des Lichtbands kontaktiert und hierdurch einen elektrischen Schlag erleidet.

Vorzugsweise ist das erfindungsgemäße Kopplungselement mit Rast- oder Schnappmitteln ausgebildet, um ein lösbares mechanisches Verbinden mit den Mitteln zur Personenerdung zu ermöglichen. Beispielsweise kann der erste Anschlussabschnitt anstatt als Druckknopf mittels einer Lötfahne gebildet sein. Die Haltekraft des ersten Anschlussabschnitts liegt dabei vorzugsweise unterhalb eines vorgegebenen Grenzwerts. Dieser ist derart bemessen, dass er in etwa einer Haltekraft von Befestigungs- bzw. Montagemitteln des Gehäuses oder Trägerelements des Geräts entspricht. D. h. selbst wenn über das Erdungsmittel ein Zug auf das sonstige Kopplungselement ausgeübt wird, besteht nicht die Gefahr, dass dieses oder darüber ein Teil des Geräts unbeabsichtigt aus seiner Verankerung beispielsweise an der Decke eines Raums gerissen wird.

Weitere Merkmale und Vorteile der Erfindung ergeben sich aus der nachfolgenden Beschreibung bevorzugter Ausführungsformen. Es zeigen:
- Figur 1: ein Gerät mit einem Anschluss zur Energieversorgung,
- Figur 2: ein zu Figur 1 abgewandeltes Gerät mit einer daran über ein Kopplungselement gemäß einer ersten Ausführungsform der Erfindung geerdeten Person,
- Figur 3: den in Figur 2 gezeigten Anschluss,
- Figur 4: den Anschluss von Figur 3 in größerem Detail,
- Figur 5: eine Abwandlung des Anschlusses von Figur 3 in zwei Ansichten,
- Figur 6: eine Abwandlung des Anschlusses von Figur 5,
- Figur 7: ein Kopplungselement gemäß einer zweiten Ausführungsform der Erfindung.

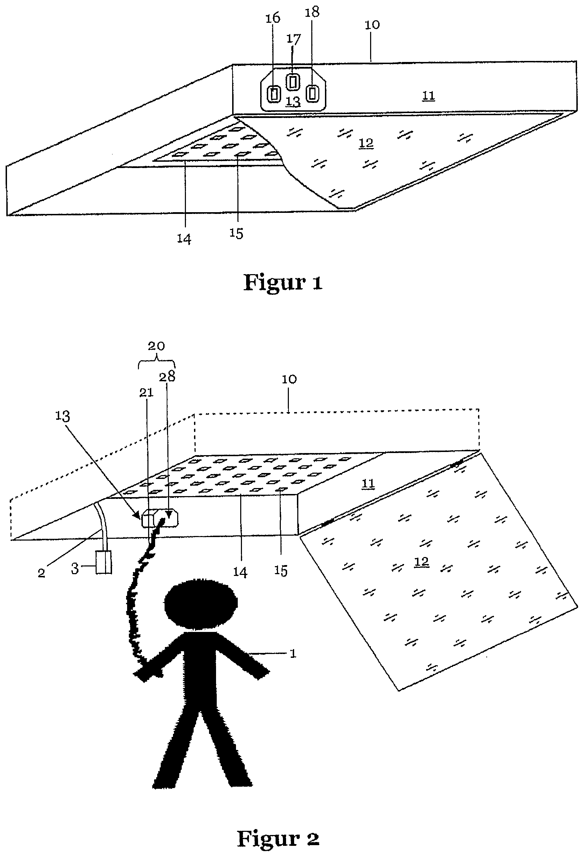

Das erfindungsgemäße Konzept soll nachfolgend anhand des Beispiels einer Leuchte 10 erläutert werden. Wie in Figur 1 gezeigt, ist die Leuchte 10 exemplarisch zum Deckenanbau vorgesehen und weist ein etwa quadratisches, topfartig ausgebildetes Leuchtengehäuse 11 auf. Die Leuchte 10 ist also als Deckenleuchte Aufputz-montiert.

Das Gehäuse 11 weist an seiner nach unten offenen Seite eine Lichtaustrittsöffnung auf, die von einer transparenten Scheibe als Abdeckung 12 zur Lichtabgabe abgedeckt ist. Das möglicherweise aus Metall bzw. Blech bestehende Leuchtengehäuse 11 ist dabei mittels nicht näher dargestellter Befestigungselemente an der Decke eines Raums angebracht und vorzugsweise mit dieser zur Erdung elektrisch gekoppelt. Ferner kann das Gehäuse 11 in anderer Weise geerdet sein.

Das Gehäuse 11 weist an seiner hier nach vorne weisenden, exemplarischen Außenseite einen Energieversorgungsanschluss als Kopplungsabschnitt in Form einer Kaltgeräte-Anschlussbuchse 13 auf. Die Anschlussbuchse 13 ist frei zugänglich und dient primär dem Zweck, das Gerät 10 mit Energie zu versorgen. Die Anschlussbuchse 13 weist demnach bekanntermaßen einen Stromanschluss 16, einen Nullleiteranschluss 17 und einen Neutrallleiteranschluss 18 auf. Die Anschlüsse 16, 18 dienen der eigentlichen Energieversorgung. Der Nullleiteranschluss bzw. Erdungsanschluss 17 ist zum Erden der Leuchte 10 vorgesehen. Ist deren Gehäuse 11 aus Metall gefertigt, muss dieses mit dem Nullleiteranschluss 17 elektrisch gekoppelt sein.

Ferner enthält die Leuchte 10 LEDs als Leuchtmittel 15, von denen der Übersichtlichkeit wegen nur eine mit Bezugszeichen versehen ist. Speziell die Leuchtmittel 15 bzw. exemplarisch hier ein diese tragendes LED-Modul 14 muss jederzeit vor elektrostatischen Entladungen geschützt sein. Die LEDs 15 sind beispielhaft matrixartig auf einer oder mehreren Leiterplatten angeordnet. Die Leiterplatten sind an einer hier nach unten weisenden Bodenseite des Gehäuses 11 befestigt und mit nicht näher dargestellten Mitteln zur Stromversorgung, also beispielsweise einem Konverter oder dergleichen, elektrisch gekoppelt. Hier gibt es exemplarisch eine Leiterplatte, die mit den darauf angebrachten LEDs 15 und etwaig anderen vorgesehenen elektronischen Bauelementen, inklusive deren Verschaltung, das Modul 14 bildet. Bei den LEDs 15 handelt es sich um elektronische Komponenten, die insbesondere im Hinblick auf unerwünschte elektrostatische Entladungen äußerst sensibel sind. Derartige Entladungen können zu einer Beschädigung der LEDs 15 führen, die sich möglicherweise nicht unmittelbar auswirkt, allerdings zu einem späteren Zeitpunkt möglicherweise zu einem Totalausfall der Leuchtmittel 15 führen kann. Dabei besteht insbesondere die Gefahr einer unkontrollierten und damit möglicherweise zu hohen entsprechenden Entladung über die LEDs 15, wenn eine nicht ordnungsgemäß geerdete, hier nicht dargestellte Person 1 beispielsweise die Leiterplatte berührt.

Um vor Ort, also bspw. im an einer Decke eines Raums montierten Zustand der Leuchte 10, Reparatur- oder Wartungsarbeiten durchführen zu können und trotz allem den erforderlichen ESD-Schutz einzuhalten, kann im Inneren des Leuchtengehäuses 11, beispielsweise an einer Seitenwand hiervon, ein Entladewiderstand mit dem Nullleiteranschluss 17 elektrisch gekoppelt sein. Ist das Gehäuse 11 insgesamt geerdet, bedeutet dies, dass der Anschluss 13 über den hier mittig angeordneten Nullleiteranschluss 17 hier mit einer Wandung des Gehäuses 11 elektrisch gekoppelt ist.

Figur 2 zeigt eine zu Figur 1 abgewandelte Leuchte 10 mit einer daran über ein Kopplungselement 20 gemäß einer ersten Ausführungsform der Erfindung geerdeten Person 1.

Die Leuchte 10 ist hier Unterputz verbaut. D. h. das hier wiederum nach unten offene Gehäuse 11 ist in eine nicht weiter dargestellte Decke versenkt. Im in Figur 1 gezeigten Zustand ist also nur die Abdeckung 12 sichtbar. Die Abdeckung 12 hat hier die Form einer Plexiglasscheibe, die über nicht bezeichnete Scharniere am Gehäuse 11 angelenkt und in Richtung offene Seite von der Raumdecke weg verschwenkt ist. Auch hier enthält die Leuchte 10 im Gehäuse 11 und nach unten weisend Leuchtmittel 15 wiederum exemplarisch in Form von LEDs in einem Leuchtmodul 14.

Die Leuchte 10 verfügt exemplarisch wieder über eine Kaltgerätanschlussbuchse 13, die hier innenseitig am Gehäuse 11 angeordnet ist. D. h. bei geschlossener Abdeckung 12 ist die Anschlussbuchse 13 nicht zugänglich und vorteilhafterweise auch nicht sichtbar. Die Energieversorgung der Leuchte 10 erfolgt über einen korrespondierenden Kaltgerätestecker als Steckverbinder 3, der mit einem Energieversorgungskabel 2 elektrisch gekoppelt ist. Das Kabel 2 ist in bekannter Weise als Netzspannungskabel ausgebildet.

Im in Figur 2 gezeigten Zustand ist der Steckverbinder 3 ausgesteckt, sodass die Leuchte 10 stromlos ist. Die Person 1 bzw. exemplarisch deren Handgelenk ist über ein Erdungsmittel 21 (hier bspw. ein Erdungskabel 21, wie einem Antistatikband) mit einem Anschluss 28, hier in Form eines Kaltgerätesteckers, elektrisch gekoppelt. Der Anschluss 28 ist also zur Anschlussbuchse 13 korrespondierend ausgebildet und hier in diesen in bekannter Weise eingesteckt. Der hier nicht sichtbare Nullleiteranschluss 17 der Anschlussbuchse 13 ist geerdet, obwohl der Steckverbinder 3 ausgesteckt ist. Dies bedeutet, dass die Person 1 über das Erdungskabel 21 geerdet ist und damit weder gefährdet ist, Stromschläge zu erleiden, noch in der Lage ist, aufgrund etwaiger eigener statischer Aufladung elektronisch sensible Teile der Leuchte 10 zu beschädigen. D. h der Energieversorgungsanschluss 13 des Geräts 10 kann genutzt werden, die Person 1 über das Gerät 10 zu erden, sodass diese Arbeiten an der Leuchte 10 durchführen kann und dementsprechend eine definierte Entladung über einen im Erdungskabel 21 und/oder im Anschluss 28 vorsehbaren Entladewiderstand vorgenommen werden kann.

Das Erdungskabel 21 ist vorzugsweise mit dem Anschluss 28 des Kopplungselements 20 einstückig ausgebildet. Dies bedeutet, dass die Person 1 unter Einhaltung der ESD-Vorschriften nur mit dem Gerät 10 elektrisch gekoppelt werden kann, wenn der Anschluss 28 angeschlossen ist. Dies verhindert die Gefahr, dass sich die Person 1 über einen etwaig anderen, extra vorhandenen Erdungsanschluss 10 am Gerät 10 erden kann und dieses unter Strom stehen könnte. Mithin erhöht sich die Bediensicherheit.

Figur 2 verdeutlicht zudem ein mit der Erfindung verbundenes Verfahren. Nachdem die die Arbeiten durchführende Person 1 die Leuchte 10 durch Entfernen oder beispielsweise seitliches Verschwenken der Abdeckscheibe 12 geöffnet hat, stellt sie eine elektrisch leitende Verbindung zur Anschlussbuchse 13 des Geräts 10 her; und zwar hier über den Anschluss 28. Die elektrische Kopplung erfolgt, wie beschrieben, über das Erdungskabel 21, das am Handgelenk der Person 1 befestigt und über den Anschluss 28 als Kopplungsmittel mit dem Gerät 10 elektrisch gekoppelt wird. Durch diese elektrische Kopplung erfolgt also über den vorteilhafterweise ebenfalls vorhandenen Entladewiderstand eine elektrische Kopplung mit dem Nulleiteranschluss 17 des Stromanschlusses 13 und damit mit einem nicht weiter dargestellten Erdungsanschluss der Leuchte 10. Dadurch ist die Person 1 nunmehr geerdet, und eventuelle Ladungen können definiert über den Entladewiderstand abgeführt werden. Zudem ist das Gerät 10 im gezeigten Beispiel von einer externen Energieversorgung getrennt, sodass das Gerät 10 selbst stromlos sein kann und daher keine Gefahr für die Person 1 besteht, über das Gerät 10 einen Stromschlag oder dergleichen zu erleiden. Bleibt die Person 1 weiterhin auf diese Weise mit dem Leuchtengehäuse 11 elektrisch gekoppelt, besteht während der nachfolgend durchzuführenden Arbeiten also weder die Gefahr der elektrostatischen Aufladung, sodass der ESD-Schutz beibehalten bleibt, noch die einer Verletzung aufgrund fließender Ströme im Gerät 10.

Die Kopplung zwischen Erdungskabel 21 und Anschluss 28 bzw. zwischen Anschluss 28 und Anschlussbuchse 17 ist dabei derart bemessen, dass eine zum Lösen der elektrischen Kopplung erforderliche Kraft eine zulässige Maximallast der Leuchte 10 nicht überschreiten kann. Die Maximallast der Leuchte 10 bemisst sich dabei insbesondere nach der Tragkraft der Befestigungsmittel, mit deren Hilfe die Leuchte 10 an der Decke des Raums montiert ist. Sollte also beispielsweise die Person 1 stürzen, so besteht nicht die Gefahr, dass die Leuchte 10 von der Decke gerissen wird, da sich stattdessen zuvor die elektrische Kopplung zwischen Erdungskabel 21 und Gerät 10 lösen wird.

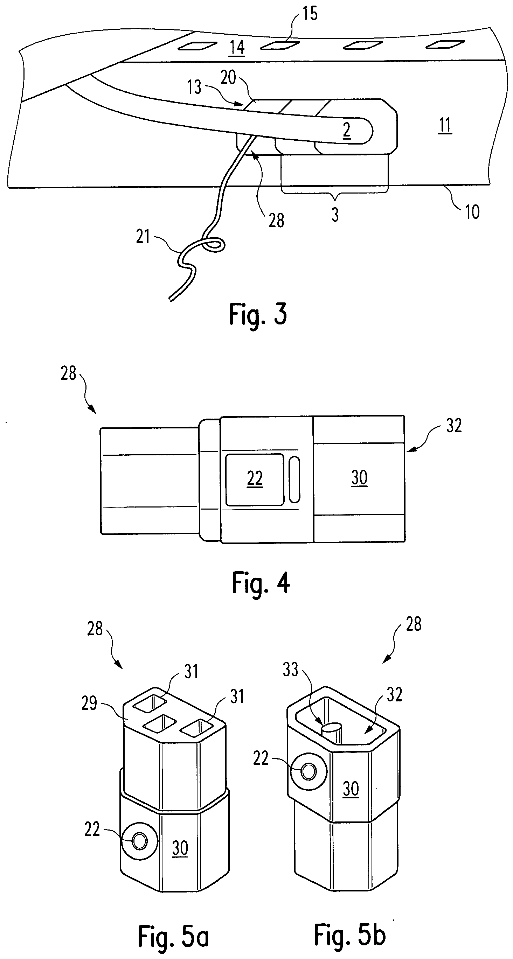

Figur 3 zeigt eine Abwandlung zur in Figur 2 gezeigten Ausführungsform im Ausschnitt und in größerem Detail. Die Leuchte 10 ist exemplarisch gemäß Figur 2 ausgebildet. Sie weist also ein nach unten offenes Gehäuse 11 mit Leuchtmodul 14 und LEDs 15 auf. Allerdings ist der Anschluss 28 des Kopplungselements 20 hier nicht nur ausgebildet, in die hier ebenfalls verdeckt angeordnete Anschlussbuchse 13 eingesteckt zu werden. Zudem ist er hier an seiner der Anschlussbuchse 13 abgewandten Seite gestaltet, dass der Steckverbinder 3 aufgesteckt werden kann, wie er auch auf die Anschlussbuchse 13 aufgesteckt würde. Dazu kann er genauso wie die Anschlussbuchse 13 ausgebildet sein. Dies ermöglicht es, die Leuchte 10 weiterhin mit Energie versorgen zu können, obwohl die hier nicht abgebildete Person 1 Wartungsarbeiten durchführt. Dies kann beispielsweise notwendig sein, wenn Betriebstests durchgeführt werden müssen. Und trotzdem sind die elektronischen Elemente der Leuchte 10 geschützt.

Das Erdungskabel 21 des Kopplungselements 20 ist exemplarisch an dem Anschluss 28 angesteckt und somit separat ausgebildet. Aufgrund der Nutzung des Nullleiters des Kabels 2 ist die Person 1 geerdet und vor etwaigen Stromschlägen geschützt.

Figur 4 zeigt den Anschluss 28 des sonst nicht weiter dargestellten Kopplungselements 20 von Figur 3 in einer Seitenansicht. Der Anschluss 28 weist ein Gehäuse 30 auf, an dessen hier rechter Seite eine nicht sichtbare, nach rechts offene Ausnehmung 32 ausgebildet ist. Beispielhaft mittig weist der Anschluss 28 einen Erdungsanschluss bzw. Anschlussabschnitt beispielsweise in Form eines Druckknopfanschlusses 22 auf. D. h. das hier nicht abgebildete Erdungskabel 21 verfügt an seinem der Person 1 entfernten Ende über einen zum Druckknopfanschluss 22 korrespondierenden Druckknopfanschluss. Dies hat den Vorteil, ein bereits vorhandenes Erdungskabel 21 weiterhin nutzen zu können. Es muss lediglich der Anschluss 28 beschafft werden, was die Herstellungs- und Beschaffungskosten gering hält.

Figur 5 zeigt eine Abwandlung des Anschlusses 28 von Figur 4 in zwei verschiedenen perspektivischen Ansichten. Figur 5a zeigt den Anschluss 28 mit einer Sicht auf die in Figur 4 linke Stirnseite, und Figur 5b auf die rechte.

Wie in Figur 5a gezeigt, ist der Anschluss 28 an der hier nach oben weisenden Seite wie eine Kaltgerätebuchse ausgebildet. Er weist mithin zwei äußere Anschlussbuchsen 31 für die Energieversorgungsanschlüsse des Steckverbinders 13 und mittig einen Erdungsanschluss 29 für den Nullleiteranschluss des Steckverbinders 13 auf. Der Steckverbinder 3 kann also auf diese Seite des Anschlusses 28 aufgesteckt und dabei automatisch mit seinen drei Anschlüssen oder zumindest mit seinem mittigen Nullleiteranschluss mit den/m korrespondierenden Anschlussbuchsen 29, 32 bzw. der korrespondierenden Anschlussbuchse 29 des Anschlusses 28 elektrisch gekoppelt werden.

Wie in Figur 5b gezeigt, ist der Anschluss 28 an der in Figur 5a nach unten weisenden Stirnseite wie ein Kaltgerätestecker ausgebildet, allerdings in abgewandelter Form. Anstelle üblicherweise dreier Anschlussstecker ist hier nur in der Mitte ein Kopplungsabschnitt bzw. Anschlussstecker 33 ausgebildet. Der Anschlussstecker 33 ist dabei in der Ausnehmung 32 freistehend ausgebildet. Die Ausnehmung 32 ist gemäß einem üblichen Kaltgerätestecker ausgebildet. Wird der Anschluss 28 mit dieser Stirnseite bzw. Ausnehmung 32 auf die Anschlussbuchse 13 des Geräts 10 aufgesteckt, erfolgt sowohl eine mechanische Fixierung des Anschlusses 28 an der Buchse 13 als auch eine elektrische Kopplung zwischen Anschlussstecker 33 und Nullleiteranschluss 17, und zwar nur zwischen diesen beiden Anschlüssen 33, 17.

Der Anschlussstecker 33, der Erdungsanschluss 29 und der Druckknopfanschluss 22 sind über eine im Gehäuse 30 vorgesehene Verschaltung miteinander elektrisch gekoppelt. Koppelt sich die Person 1 also über ein Erdungskabel 21 elektrisch mit dem Anschluss 28, und wird dieser sozusagen zwischen den Steckverbinder 3 und die Anschlussbuchse 13 geschaltet, so ist die Person 1 automatisch geerdet und kann selbst aufgrund etwaiger elektrostatischer Aufladung keine elektronisch sensiblen Teile des Geräts 10 beschädigen. Es reicht mithin ein klassischer Energieversorgungsanschluss (mit Nullleiteranschluss) des Geräts 10 aus; zusätzliche Vorkehrungen müssen am Gerät 10 nicht vorgesehen werden.

Der Druckknopfanschluss 22 ist in dem Bereich angeordnet, in dem sich auch die Ausnehmung 32 befindet, also im Bereich des Kaltgerätesteckers, da der Bereich der Kaltgerätebuchse im Einsteckzustand mit dem Steckverbinder 3 von außen nicht zugänglich wäre.

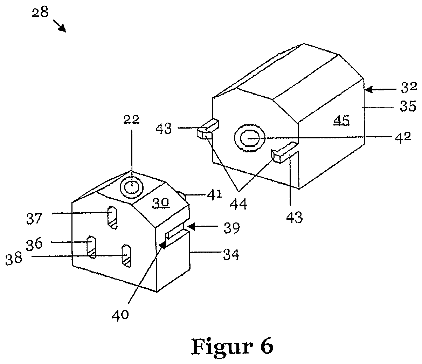

Figur 6 zeigt eine Abwandlung des Anschlusses 28 von Figur 5. Wie zu erkennen, ist der Anschluss 28 zweiteilig ausgebildet. Er umfasst zwei Anschlussteile 34, 35. Jedes Anschlussteil 34, 35 verfügt im gezeigten Beispiel über ein jeweiliges Gehäuse 30 bzw. 45.

Das hier linke Anschlussteil 34 ist an seiner dem Anschlussteil 35 abgewandten, hier nach vorne weisenden Seite beispielhaft analog der Anschlussbuchse 13 gestaltet. D. h. das Anschlussteil 34 weist an dieser Seite eine Stromanschlussbuchse 36, eine Nullleiteranschlussbuchse 37 und eine Neutralleiteranschlussbuchse 38 auf. Hier oberseitig ist noch ein Druckknopfanschluss 22 zum Anschluss eines nicht dargestellten Erdungskabels 21 vorgesehen. An seiner dem Anschlussteil 35 zugewandten Seite steht vom Gehäuse 30 in Richtung ebenjenem Anschlussteil 35 ein Kopplungs- bzw. Steckerabschnitt 41 aus elektrisch leitfähigem Material wie Metall hervor. Die Nullleiteranschlussbuchse 37, der Druckknopfanschluss 22 und der Steckerabschnitt 45 sind über eine im Gehäuse 30 vorhandene Verschaltung untereinander elektrisch gekoppelt.

Das Gehäuse 30 verfügt ferner über Befestigungsabschnitte. Diese sind hier an zwei einander gegenüberliegenden Außenseiten ausgebildet, von denen nur die vordere sichtbar ist. Jeder Befestigungsabschnitt umfasst eine Führungsnut 39, die sich von der dem Anschlussteil 35 zugewandten Seite her in Richtung der Seite mit den Kopplungsabschnitten bzw. Buchsen 36 - 38 erstreckt, aber im gezeigten Beispiel nicht durchgehend ausgebildet ist. An dem dem Anschlussteil 35 abgewandten Ende jeder Führungsnut 39 befindet sich jeweils eine Rastausnehmung 40. Jede Rastausnehmung 40 liegt, in Bezug auf die jeweilige Seite des Gehäuses 30, an der sie ausgebildet ist, tiefer als die sich daran anschließende, sonstige Führungsnut 39.

Das Anschlussteil 35 weist an seiner dem Anschlussteil 34 zugewandten Seite einen Kopplungs- bzw. Buchsenabschnitt 42 und zwei Führungsvorsprünge 43 auf. An seiner dem Anschlussteil 34 abgewandten Seite ist das Anschlussteil 35 exemplarisch analog Figur 5b mit einer Ausnehmung 32 versehen, in der zumindest mittig ein hier nicht sichtbarer Steckerabschnitt 33 ausgebildet ist. Der Steckerabschnitt 33 ist über eine interne Verschaltung mit dem Buchsenabschnitt 42 elektrisch gekoppelt.

Die Führungsvorsprünge 43, 43 stehen vom Anschlussteil 35 in Richtung Anschlussteil 34 hervor und verlaufen beispielsweise parallel zueinander. Sie haben zueinander einen Abstand, der im Wesentlichen einem Abstand der beiden Seiten des Anschlussteils 34 mit den Führungsnuten 39, verringert um deren maximale Nutentiefen, entspricht.

An ihren freien Enden weisen die Führungsvorsprünge 43 jeweils einen Rastvorsprung 44 auf. Die Rastvorsprünge 44, 44 erstrecken sich dabei aufeinander zu und haben mithin zueinander einen geringeren Abstand als die sonstigen Führungsvorsprünge 43, 43 zueinander.

Die Rastvorsprünge 44, 44 weisen an ihren dem Anschlussteil 35 abgewandten Seiten vorzugsweise Anlaufschrägen auf. Werden die Anschlussteile 34, 35 aufeinander zu bewegt, kommen die Rastvorsprünge 44, 44 mit den Anlaufschrägen mit einer jeweiligen Nut 39 in Anlage und werden beim weitergehenden Aufeinanderzubewegen der Anschlussteile 34, 35 voneinander weg gedrängt. Erreichen sie die jeweilige Rastausnehmung 40, schnappen sie in diese zurück, sodass die Anschlussteile 34, 35 aneinander fixiert sind. Sind die Anschlussteile 34, 35 mit ihren einander zugewandten Seiten zusammengeschoben, gelangen die Führungsvorsprünge 43 aufgrund des Zurückschnappens der Rastvorsprünge 44, 44 mit ihren einander zugewandten Seiten mit einer jeweiligen der Führungsnuten 39 in Anlage und schließen mit der zugehörigen Seite des Gehäuses 30 vorzugsweise bündig ab.

Bei dieser Bewegung gelangt der Steckerabschnitt 41 in den Buchsenabschnitt 42 und koppelt mit diesem elektrisch. Damit sind Nullleiteranschluss 37, Druckknopfanschluss 22 und Steckerabschnitt 33 miteinander elektrisch gekoppelt, verbunden mit der in den vorigen Ausführungsformen beschriebenen Wirkung.

Diese Ausführung hat den Vorteil, dass die hier vordere Seite des Anschlussteils 34 eine andere Anschlusskonfiguration haben kann als die an der davon abgewandten Seite des Anschlussteils 35. Auch wenn die Erfindung mit einer einzigen Anschlusskonfiguration gemäß dem Kaltgeräteanschlussstandard IEC-60320 C14 beschrieben wurde, ist daher klar, dass jede andere Anschlusskonfiguration genutzt werden, wie sie beispielsweise für 220 V-Steckdosen üblich ist. Es muss nur sichergestellt sein, dass der jeweilige Anschluss am Gerät 10 einen Erdungsanschluss beinhaltet. Beispielsweise kann diese Seite des Anschlussteils 35 zur hier nach vorne weisenden Seite des Anschlussteils 35 ausgebildet sein, also ebenfalls über drei Anschlussbuchsen verfügen. Oder aber eine der Seiten 34, 35 hat beispielsweise die Form eines Schuko-Steckers. Diese Lösung ermöglicht mithin, an sich zueinander inkompatible Anschlüsse miteinander kombinieren zu können. Zudem kann eines der Anschlussteile 34, 35 immer gleich ausgebildet sein.

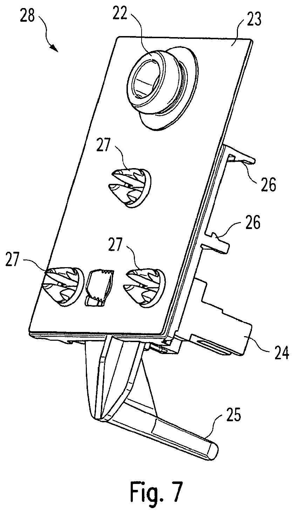

Figur 7 zeigt einen Anschluss 28 gemäß einer zweiten Ausführungsform der Erfindung. Der Anschluss 28 ist nicht wie ein externer Energieversorgungsanschluss ausgebildet. Stattdessen weist er einen hier plattenartigen Halteteil 23 auf.

Das Halteteil 23 weist an seiner hier nach vorne weisenden Seite beispielhaft wiederum einen Druckknopfanschluss 22 für den elektrischen und vorteilhafterweise mechanischen Anschluss eines nicht dargestellten Erdungskabels 21 auf, das anderenends gestaltet ist, in bekannter Weise mit einer Person 1 elektrisch gekoppelt zu werden.

An seiner dem Druckknopfanschluss 22 weg weisenden Seite steht vom Halteteil 23 hier ein Steckerabschnitt 24 hervor. Dieser ist gestaltet, mit einem korrespondierenden Anschluss des Geräts mechanisch, zumindest aber elektrisch gekoppelt zu werden. Der Steckerabschnitt bzw. Kopplungsabschnitt 24 weist einen nicht sichtbaren Erdungsanschluss auf, der beim Aufsetzen des Steckerabschnitts 24 mit einem korrespondierenden Erdungsanschluss des hier nicht abgebildeten Geräts 10 elektrisch gekoppelt wird und ferner über eine interne Verschaltung mit dem Druckknopfanschluss 22 elektrisch gekoppelt ist. Die Verschaltung kann dadurch realisiert sein, dass das Halteteil 23 mittels einer Leiterplatte gebildet ist oder solch eine aufweist. Die Leiterplatte koppelt dann den Erdungsanschluss und den Druckknopfanschluss 22 des Anschlusses 28 in bekannter Weise über eine Leiterbahn miteinander.

Der Steckerabschnitt 24 ist vorzugsweise an dem Halteteil 23 über hier drei Rastköpfe 27 mechanisch befestigt, die in die gleiche Richtung hervorstehen wie der Druckknopfanschluss 22.

Zusätzlich zum Steckerabschnitt 24 stehen hier zwei Rastnasen 26 vom Halteteil 23 hervor. Diese dienen dem Verrasten des Anschlusses 28 mit beispielsweise einer korrespondierenden Leiterplatte des nicht dargestellten Geräts 10 hier zum Zwecke des Entnehmens dieser aus ebenjenem Gerät 10. D. h. beim elektrischen Koppeln des Anschlusses 28 mit dem Gerät 10 gelangt dieser zugleich in Rasteingriff mit der hier zu entnehmenden Leiterplatte; beispielsweise einer Ansteuerschaltung für LEDs 15.

Zusätzlich ist am Halteteil 23 ein hervorstehender Vorsprung 25 ausgebildet. Das Gerät 10 weist dementsprechend eine Ausnehmung auf, die so gestaltet ist, dass der Vorsprung 25 beim Aufsetzen des Anschlusses 28 auf das Gerät 10 in der Ausnehmung geführt wird und der Anschluss 28 so in die Montageposition bewegt wird. Vorzugsweise sind die Rastnasen 26 und der Vorsprung 25 an ein und demselben Teil ausgebildet, das vorteilhafterweise noch über die Rastköpfe 27 verfügt.

Die Erfindung ist nicht auf die vorbeschriebenen Ausführungsformen beschränkt.

Die Ausführungsformen können in Teilen oder insgesamt gegeneinander ausgetauscht oder miteinander kombiniert sein. Die lösbare Ankoppelbarkeit eines Erdungskabels 21 kann bei allen Ausführungsformen zusätzlich oder alternativ zu einem fest angeschlossenen Erdungskabel 21 vorgesehen sein. Das Halteteil 23 kann vorzugsweise an der Seite des Druckknopfanschlusses 22 ebenfalls über eine Anschlussmöglichkeit nunmehr für einen Konverter oder eine sonstige Energieversorgung, Testgerät oder dergleichen aufweisen, wie im übrigen die anderen Ausführungsformen in Form des Kopplungselements 20 oder seines Anschlussteils 34 auch. Jede Ausführungsform kann zusätzliche Funktionselemente wie die Rastnasen 26 bei der zweiten Ausführungsform aufweisen.

Als elektrische Anschlussmöglichkeiten kommen anstelle oder zusätzlich zu den Kaltgeräteanschlüssen bzw. dem Steckerabschnitt 24 alle anderen Kopplungskonfigurationen wie USB, RJ44 und dergleichen in Frage. Es gibt keine Beschränkung auf die gezeigten Anschlussmöglichkeiten.

Vorstehend ist zwar hauptsächlich die Rede vom elektrischen Koppeln. Es ist allerdings klar, dass dies, wenn möglich, mit einem mechanischen Kuppeln bzw. Fixieren der miteinander elektrisch zu koppelnden Elemente einhergeht, wie dies insbesondere bei Stecker-Buchsen-Kombinationen der Fall ist.

Der Begriff "Verschaltung" ist rein funktionell gemeint, und zwar lediglich hinsichtlich der einer elektrischen Kopplung. Die tatsächliche konstruktive Ausgestaltung der Verschaltung kann auf verschiedenste Art und Weise beispielsweise mittels der in Bezug auf Figur 7 beschriebenen Leiterplattenlösung erfolgen. Bei der Ausführungsform gemäß Figur 6 könnten die Anschlussbuchse 37, der Druckknopfanschluss 22 und der Steckerabschnitt 41 in Teilen oder insgesamt einstückig miteinander ausgebildet, aneinander angelötet, miteinander verschraubt und/oder in sonstiger Weise aneinander befestigt sein. Diese so gebildete Struktur kann mit einem Kunststoff umgossen sein, sodass sich das Anschlussteil 34 bildet.

Anstelle des Anschlusses 13 kann ein Anschluss beispielsweise für eine Durchverdrahtung vorgesehen sein, mit dem Kopplungselement 20 für die vorgenannten Arbeiten temporär gekoppelt zu werden. D. h. der eigentliche externe Energieversorgungsanschluss befindet sich an einer anderen Stelle des Geräts 10 oder vielleicht auch an einem anderen Modul 14 desselben oder eines anderen Geräts 10. Der betreffende Gerätebereich, an dem die Person arbeiten möchte, kann daher weiterhin unter Strom stehen. Nur führen die vorgenannten Lösungen eben dazu, dass für die Person 1 keine Gefahr besteht.

Die Erfindung kann bei allen Geräten zum Einsatz kommen, die einen ESD-Schutz erfordern, bzw. deren Elektronik vor ungewollten Spannungen von außen geschützt werden muss, wie sie beispielsweise aufgrund elektrostatischer Aufladung entsteht, und insbesondere bei denen, bei denen zusätzlich die Gefahr bestehen kann, dass eine Person bei Berührung einen Stromschlag oder dergleichen erleiden könnte. Dies ist beispielsweise bei Geräten zur Lagerung elektronischer Komponenten bzw. Bauelemente gegeben, also beispielsweise bei automatischen Regalen. Dies bedeutet ferner, dass die beschriebenen LEDs 15 durch völlig andere elektronische Bauelemente oder eine Elektronik (als Schaltung mit mehreren elektronischen Bauelementen) ersetzt oder mit diesen kombiniert sein können.

Die Montage der vorbeschriebenen Leuchten 10 dient nur zur Erläuterung der besonderen Eignung der Erfindung für Leuchten. Die Montageart ist für die Erfindung insofern unerheblich, solange eine Anschlussmöglichkeit für das Kopplungselement 20 besteht. Beispielsweise könnte die Leuchte 10 in einem Rahmengestell untergebracht sein, das beispielhaft deckenseitig montiert ist. In dem Fall könnte der geräteseitige Anschluss für das Kopplungselement 20 auch am Rahmengestell vorgesehen sein und nicht an der Leuchte 10.

Das Rahmengestell mit der/n Leuchte/n 10 würde im Rahmen der Erfindung dann zum Gerät.

Anstelle der Nutzung des Nullleiters kann der Energieversorgungsanschluss des Geräts selbstverständlich einen extra Erdungsanschluss beinhalten. Demzufolge hat das Kopplungselement 20 damit korrespondierend ebenfalls einen extra Erdungsanschluss. Dies ist dann besonders wichtig, wenn es keinen Nullleiteranschluss am Gerät 10 gibt. In dem Fall sieht das Kopplungselement 20 vor, über seinen Erdungsanschluss mit dem Gerät 10 geerdet zu werden. Dies kann mittels einer Klemme erfolgen, die an das Gerät 10 geklemmt werden kann und mit dem Erdungsanschluss elektrisch gekoppelt ist.

Der vorgenannte Druckknopfanschluss 22 kann zusätzlich zu einem bereits an den Anschluss 28 angeschlossenen Erdungskabel 21 vorgesehen sein. Dies ermöglicht, zwei Personen gleichzeitig zu erden. Der Anschluss 28 kann dabei an eine Art Standgerät angeschlossen sein, an dem die Erdungskabel 21 anzuschließen sind.

Die Rastmittel 40, 41 können durch jede andere Art der Befestigung ersetzt oder mit dieser kombiniert sein. Beispielsweise bietet sich eine Klemmung an, wie sie bei üblichen SteckerBuchsen-Kombinationen zur Anwendung kommt. Die Anschlussbuchsen 36 - 38 können entfallen, sodass nur das Anschlussteil 34 auf das Anschlussteil 35 aufgesetzt werden kann, nichts anderes aber auf das Anschlussteil 34, sodass eine Konfiguration gemäß beispielsweise Figur 2 oder Figur 6 entsteht.

Anstelle zweier Anschlussteile 34, 35 können auch drei Anschlussteile vorgesehen sein, die vorzugsweise ineinander steckbar, zumindest aber miteinander elektrisch koppelbar sind. Das Mittelteil hat somit zu beiden Seiten Standard-Anschlüsse. Die anderen zwei Anschlussteile sind anschlussmäßig an den jeweiligen Einsatzort angepasst ausgebildet. Damit kann das Mittelteil den Anschluss für das Erdungskabel beinhalten oder dieses als integralen Bestandteil aufweisen. Die äußeren Anschlussteile sind lediglich Anschlussadapter.

Die Erläuterungen verdeutlichen also, dass mit Hilfe der erfindungsgemäßen Maßnahmen in sehr einfacher und eleganter Weise auch unmittelbar vor Ort die Vorschriften bzgl. eines ESD-Schutzes eingehalten werden können und zusätzlich eine Person 1 vor Verletzungen infolge möglicher Stromflüsse im Gerät 10 geschützt werden kann. Während es früher erforderlich war, die Leuchte 10 zu demontieren und zu Reparaturarbeiten an den Hersteller zurückzuschicken, können nunmehr entsprechende Maßnahmen unmittelbar vor Ort durchgeführt werden, was deutliche Vorteile hinsichtlich des Zeit- und Kostenaufwands mit sich bringt.

Im Ergebnis bietet sich die erfindungsgemäße Lösung insbesondere in solchen Fällen an, in denen eine entsprechende Steckbuchse zur lösbaren Kopplung beispielsweise eines Konverters vorgesehen ist. Das gleiche Prinzip könnte grundsätzlich allerdings auch in jeder Leuchte zum Einsatz kommen, wobei dann eben eine lösbare Verbindung des Lampenbetriebsgeräts mit einer entsprechenden Anschlussmöglichkeit zumindest für eine Erdung vorgesehen sein muss.

### Bezugszeichenliste

- 1: Person
- 2: Kabel
- 3: Steckverbinder

- 10: Leuchte
- 11: Gehäuse
- 12: Abdeckung
- 13: Anschlussbuchse
- 14: Leuchtmodul
- 15: Leuchtmittel
- 16: Stromanschluss
- 17: Nulleiteranschluss
- 18: Neutralleiteranschluss

- 20: Kopplungselement
- 21: Erdungskabel
- 22: Druckknopfanschluss
- 23: Halteteil
- 24: Steckerabschnitt
- 25: Führungsabschnitt
- 26: Rastnase
- 27: Rastkopf
- 28: Anschluss
- 29: Erdungsanschluss
- 30: Gehäuse
- 31: Anschlussbuchse
- 32: Ausnehmung
- 33: Anschlussstecker
- 34: Anschlussteil
- 35: Anschlussteil
- 36: Stromanschlussbuchse
- 37: Nullleiteranschlussbuchse
- 38: Neutralleiteranschlussbuchse
- 39: Führungsnut
- 40: Rastausnehmung
- 41: Steckerabschnitt
- 42: Buchsenabschnitt
- 43: Führungsvorsprung
- 44: Rastvorsprung
- 45: Gehäuse

## Patentansprüche

1. Kopplungselement (20), aufweisend
• einen ersten Kopplungsabschnitt (24, 33), gestaltet, mit einem ersten Erdungsanschluss (29) an einen zweiten Erdungsanschluss (17) eines zweiten Kopplungsabschnitts (13) eines elektrisch betriebenen Geräts (10) elektrisch angeschlossen zu werden, welcher zweite Kopplungsabschnitt (13) zum elektrischen Anschluss einer elektrischen Energieversorgung für das Gerät (10) ausgebildet ist, und
• ein Erdungsmittel (21) mit einem ersten Anschlussabschnitt, der
- mit dem ersten Erdungsanschluss (29) elektrisch gekoppelt ist und
- ausgebildet ist, unmittelbar mit einer Person (1) elektrisch gekoppelt zu werden
• ein erstes Anschlussteil (35, 34) mit
• dem ersten Kopplungsabschnitt (24, 33),
• einem dritten Kopplungsabschnitt (42, 41) und
• einem Verbindungsabschnitt sowie
• ein zweites Anschlussteil (34, 35) mit einem vierten Kopplungsabschnitt (41, 42),
• wobei der Verbindungsabschnitt derart mit dem ersten und dritten Kopplungsabschnitt (24; 42, 41) elektrisch gekoppelt ist und der dritte Kopplungsabschnitt (42, 41) derart mit dem vierten Kopplungsabschnitt (41, 42) elektrisch koppelbar gestaltet ist, dass bei einem elektrischen Anschließen des zweiten Anschlussteils (34, 35) über das erste Anschlussteil (35, 34) an das Gerät (10) der erste Erdungsanschluss (29) mit dem zweiten Erdungsanschluss (17) elektrisch gekoppelt ist, wobei
• der dritte Kopplungsabschnitt (42, 41) als Stecker oder Buchse ausgebildet ist und
• der vierte Kopplungsabschnitt (41, 42) als Buchse bzw. Stecker ausgebildet ist, die bzw. der so ausgebildet ist, dass der erste und der dritte Kopplungsabschnitt (42, 41; 41, 42) derart ineinander steckbar sind, dass dadurch automatisch der erste Erdungsanschluss (29) mit dem elektrischen Verbindungsabschnitt elektrisch gekoppelt ist.

2. Kopplungselement (20) gemäß einem der vorherhergehenden Ansprüche, wobei der erste Anschlussabschnitt über einen Entladewiderstand mit dem ersten Erdungsanschluss (29) elektrisch gekoppelt ist.

3. Kopplungselement (20) gemäß Anspruch 1 oder 2, wobei das Erdungsmittel (21) einen zweiten Anschlussabschnitt aufweist, gestaltet, mit einem dritten Anschlussabschnitt (22) des Kopplungselements (20) lösbar elektrisch gekoppelt zu werden, der mit dem ersten Erdungsanschluss (29) elektrisch gekoppelt ist.

4. Kopplungselement (20) gemäß Anspruch 3, wobei der zweite Anschlussabschnitt und der dritte Anschlussabschnitt (22) mittels zweier miteinander korrespondierender, ineinander steckbarer Druckknopfanschlüsse (22) gebildet sind.

5. System, umfassend
• ein elektrisch betriebenes Gerät (10) mit einem zweiten Kopplungsabschnitt (13), der zum elektrischen Anschluss einer Stromversorgung für das Gerät (10) ausgebildet ist, und
• ein Kopplungselement (20) gemäß einem der vorhergehenden Ansprüche.

6. System gemäß Anspruch 5, wobei
. der zweite Kopplungsabschnitt (13) als Stecker oder Buchse ausgebildet ist,
. der mit dem zweiten Kopplungsabschnitt (13) elektrisch zu koppelnde erste bzw. der dritte Kopplungsabschnitt (24, 33) als Buchse bzw. Stecker ausgebildet ist, die bzw. der so ausgebildet ist, dass der zweite und der damit elektrisch zu koppelnde Kopplungsabschnitt (13) derart ineinander steckbar sind, dass dadurch automatisch der zweite Erdungsanschluss (17) mit dem ersten Erdungsanschluss (29) bzw. dem elektrischen Verbindungsabschnitt elektrisch gekoppelt ist.

7. System gemäß Anspruch 5 oder 6, wobei das Kopplungselement (20) ferner einen fünften Kopplungsabschnitt (36-38) aufweist, der gemäß dem zweiten Kopplungsabschnitt (13) des Geräts (10) ausgebildet ist und einen dritten Erdungsanschluss aufweist, der mit dem ersten Erdungsanschluss (29) elektrisch gekoppelt ist.

8. System gemäß Anspruch 7, wobei der fünfte Kopplungsabschnitt (36-38) ferner gestaltet ist, Stromanschlüsse (16, 18) des zweiten Kopplungsabschnitts (13) nach außen in Bezug auf das Gerät (10) elektrisch zu isolieren.

## Claims

1. A coupling element (20), having
• a first coupling section (24, 33), which is configured to be electrically connected to a first ground connection (29) at a second ground connection (17) of an electrically operated device (10), which second coupling section (13) is designed for the electrical connection of an electrical power supply for the device (10), and
• a grounding means (21) comprising a first connection section which
- is electrically coupled to the first ground connection (29) and
- is designed to be electrically coupled directly to a person (1)
• a first connection part (35, 34) with
- the first coupling section (24, 33),
- a third coupling section (42, 41) and
- a connection section as well as
• a second connection part (34, 35) with a fourth coupling section (41, 42),
• wherein the connection section is electrically coupled to the first and third coupling section (24; 42, 41) and the third coupling section (42, 41) is designed to be capable of being electrically coupled to the fourth coupling section (41, 42) in such a manner that in the case of an electrical connection of the second connection part (34, 35) via the first connection part (35, 34) to the device (10) of the first ground connection (29), the first ground connection (29) is electrically coupled with the second ground connection (17), wherein
• the third coupling section (42, 41) is designed as a plug or bush and
• the fourth coupling section (41, 42) is designed as a bush or plug, which is designed so that the first and the third coupling section (42, 41; 41, 42) can be plugged into one another, in such a manner that the first ground connection (29) is thus automatically electrically coupled with the electrical connection section.

2. A coupling element (20) according to any one of the preceding claims, wherein the first connection section is electrically coupled via a discharge resistor with the first ground connection (29).

3. A coupling element (20) according to Claim 1 or 2, wherein the grounding means (21) has a second connection section, designed to be detachably electrically coupled with a third connection section (22) of the coupling element (20), which is electrically coupled with the first ground connection (29).

4. A coupling element (20) according to Claim 3, wherein the second connection section and he third connection section (22) are formed by means of two mutually corresponding pushbutton connections (22) which are pluggable into each other.

5. A system, comprising
• an electrically operated device (10) with a second coupling section (13), which is designed for the electrical connection of a power supply for the device (10), and
• a coupling element (20) according to any one of the preceding claims.

6. A system according to Claim 5, wherein
• the second coupling section (13) is designed as a plug or bush,
• the first or the third coupling section (24, 33) to be electrically coupled with the second coupling section (13) is designed as a bush or plug, which is designed such that the second and the coupling section (13) to be electrically connected therewith can be plugged into each other in such a manner that the second ground connection (17) is thus automatically electrically coupled with the first ground connection (29) or the electrical connection section.

7. A system according to Claim 5 or 6, wherein the coupling element (20) additionally has a fifth coupling section (36-38), which is designed according to the second coupling section (13) of the device (10) and has a third ground connection, which is electrically coupled with the first ground connection (29).

8. A system according to Claim 7, wherein the fifth coupling section (36-38) is additionally designed to electrically insulate current connections (16, 18) of the second coupling section (13) outwardly with respect to the device (10).

## Revendications

1. Élément de couplage (20) comprenant
• une première portion de couplage (24, 33) conçu pour être connectée électriquement, avec une première connexion de mise à la terre (29), à une deuxième connexion de mise à la terre (17) d'une deuxième portion de couplage (13) d'un appareil électrique (10), cette deuxième portion de couplage (13) étant conçue pour la connexion électrique d'une alimentation en énergie électrique de l'appareil (10) et
• un moyen de mise à la terre (21) avec une première portion de connexion qui
- est couplé électriquement avec la première connexion de mise à la terre (29) et
- est conçu pour être couplé électriquement directement avec une personne (1)
• une première partie de connexion (35, 34) avec
• la première portion de couplage (24, 33),
• une troisième portion de couplage (42, 41) et
• une portion de liaison ainsi que
• une deuxième partie de connexion (34, 35) avec une quatrième portion de couplage (41, 42),
• la portion de liaison étant couplée électriquement avec les première et troisième portions de couplage (24; 42, 41) et la troisième portion de couplage (42, 41) étant pour pouvoir être couplée électriquement avec la quatrième portion de couplage (41, 42) de façon à ce que, dans le cas d'une connexion électrique de la deuxième partie de connexion (34, 35) par l'intermédiaire de la première partie de connexion (35, 34) à l'appareil (10), la première connexion de mise à la terre (29) est couplée électriquement la deuxième connexion de mise à la terre (17),
• la troisième portion de couplage (42, 41) étant conçue sous la forme d'une prise mâle ou d'une prise femelle et
• la quatrième portion de couplage (41, 42) étant sous la forme d'une prise femelle respectivement d'une prise mâle, qui est conçue de façon à ce que les première et troisième portions de couplage (42, 41 ; 41, 42) pouvant être emboîtées l'une dans l'autre de façon à ce que la première connexion de mise à la terre (29) soit couplée électriquement avec la portion de liaison électrique.

2. Élément de couplage (20) selon l'une des revendications précédentes, la première portion de connexion étant couplée électriquement par l'intermédiaire d'une résistance de décharge avec la première connexion de mise à la terre (29).

3. Élément de couplage (20) selon la revendication 1 ou 2, le moyen de mise à la terre (21) comprend une deuxième portion de connexion conçue pour être couplée électriquement de manière amovible avec une troisième portion de connexion (22) de l'élément de couplage (20), qui est couplée électriquement avec la première connexion de mise à la terre (29).

4. Élément de couplage (20) selon la revendication 3, la deuxième portion de connexion et la troisième portion de connexion (22) étant formées de deux raccords à boutons de pression (22) correspondant entre eux et emboîtables l'un dans l'autre.

5. Système comprenant
• un appareil électrique (10) avec une deuxième portion de couplage (13), qui est conçue pour la connexion électrique d'une alimentation électrique pour l'appareil (10) et
• un élément de couplage (20) selon l'une des revendications précédentes.

6. Système selon la revendication 5,
• la deuxième portion de couplage (13) étant conçue sous la forme d'une prise mâle ou d'une prise femelle,
• la première, respectivement la troisième portion de couplage (24, 33), à coupler électriquement avec la deuxième portion de couplage (13) étant conçue sous la forme d'une prise femelle, respectivement d'une prise mâle, qui est conçue de façon à ce que la deuxième portion de couplage et la portion de couplage (13) à coupler électriquement avec celle-ci puissent être emboîtées l'une dans l'autre, de telle sorte que la deuxième connexion de mise à la terre (17) soit couplée électriquement de manière automatique avec la première connexion de mise à la terre (29) ou la portion de liaison électrique.

7. Système selon la revendication 5 ou 6, l'élément de couplage (20) comprenant en outre une cinquième portion de couplage (36 - 38) qui est conçue conformément à la deuxième portion de couplage (13) de l'appareil (10) et comprenant une troisième connexion de mise à la terre qui est couplée électriquement avec la première connexion de mise à la terre (29).

8. Système selon la revendication 7, la cinquième portion de couplage (36 - 38) étant en outre conçue pour isoler électriquement les connexions électriques (16, 18) de la deuxième portion de couplage (13) de l'extérieur par rapport à l'appareil (10).
